(19) Europäisches Patentamt
European Patent Office
Office européen des brevets

(11) **EP 4 287 061 A1**

(12) **EUROPEAN PATENT APPLICATION**
published in accordance with Art. 153(4) EPC

(43) Date of publication:
**06.12.2023 Bulletin 2023/49**

(51) International Patent Classification (IPC):
**G06F 30/20** (2020.01)

(21) Application number: **23718711.7**

(22) Date of filing: **31.03.2023**

(86) International application number:
**PCT/CN2023/085623**

(87) International publication number:
**WO 2023/202355 (26.10.2023 Gazette 2023/43)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.04.2022 CN 202210401552**

(71) Applicant: **China Three Gorges Corporation Wuhan, Hubei 430010 (CN)**

(72) Inventors:
• **LI, Zhou**
  **Wuhan, Hubei 430010 (CN)**
• **ZHANG, Wei**
  **Wuhan, Hubei 430010 (CN)**
• **DAI, Jialin**
  **Wuhan, Hubei 430010 (CN)**
• **LUO, Lunbo**
  **Wuhan, Hubei 430010 (CN)**

(74) Representative: **Barker Brettell LLP**
**100 Hagley Road**
**Edgbaston**
**Birmingham, West Midlands B16 8QQ (GB)**

(54) **SOIL BODY STATE DATA CALCULATION METHOD AND DEVICE BASED ON BOUNDARY SURFACE PLASTICITY MODEL**

(57) The present application discloses a method for calculating soil mass state data based on a boundary surface plasticity model, the method comprises: acquiring soil mass state data of a previous increment step; calculating a first soil mass increment based on the soil mass state data, a preset strain increment and an initial sub-increment step length; calculating a second soil mass increment from the soil mass state data and the first soil mass increment; calculating a local truncation error according to the soil mass state data, the first soil mass increment and the second soil mass increment; when the local truncation error is smaller than a preset error threshold value, obtaining soil mass state data of the current sub-increment step based on the soil mass state data of the previous increment step, the first soil mass increment and the second soil mass increment; recursive calculation is carried out in sequence until the soil mass state data of the last sub-increment step is obtained so as to obtain the soil mass state data of the current increment step.

acquiring soil mass state data of a previous increment step, wherein the soil mass state data includes stress data, strain data, and internal variable data — S1

calculating a first soil mass increment of a current sub-increment step based on the soil mass state data, a preset strain increment and an initial sub-increment step length — S2

calculating a second soil mass increment from the soil mass state data and the first soil mass increment — S3

calculating a local truncation error of the current sub-increment step according to the soil mass state data, the first soil mass increment and the second soil mass increment — S4

when the local truncation error is smaller than a preset error threshold value, obtaining soil mass state data of the current sub-increment step based on the soil mass state data of the previous increment step, the first soil mass increment and the second soil mass increment — S5

returning to the step of calculating the first soil mass increment, and calculating the soil mass state data of the next sub-increment step based on the soil mass state data of the current sub-increment step, the preset strain increment and the sub-increment step length, wherein recursive calculation is carried out in sequence until the soil mass state data of the last sub-increment step is obtained, and taking the soil mass state data of the last sub-increment step as the soil mass state data of the current increment step — S6

FIG. 1

**Description**

**CROSS REFERENCE TO RELATED APPLICATIONS**

The present application claims priority to Chinese Patent Application No. 202210401552.8 filed to CNIPA on April 18, 2022 and entitled " A METHOD AND APPARATUS FOR CALCULATING SOIL MASS STATE DATA BASED ON BOUNDARY SURFACE PLASTICITY MODEL ", the entire contents of which are incorporated herein by reference.

**TECHNICAL FIELD**

The present application relates to the technical field of rock and soil mechanics, and particularly relates to a method and apparatus for calculating soil mass state data based on boundary surface plasticity model.

**BACKGROUND**

Due to the fact that the mathematical expression of incremental constitutive relations essentially belongs to a group of ordinary differential equations, it is impossible to obtain an accurate analytical expression of the stress-strain total relationship by direct integration in most cases. Currently, there are two main types of stress integration algorithms internationally: one type is implicit integration algorithms based on the concept of regression mapping, and the other type is explicit integration algorithms based on multi-step methods. The conventional explicit integration algorithm divides the strain increments into pure elastic strain increments and elastic-plastic strain increments through iterative trial calculations to accurately locate stress reversal points. However, the boundary surface plasticity model established by considering boundary surface plasticity has no elastic domain, and all strain increments are elastic-plastic strain increments, which makes it impossible to locate stress reversal points in this manner. The boundary surface plasticity model generally judges the stress reversal by recognizing the change between positive and negative signs of a load index, but when the sub-increment step length is large, the position of the stress reversal point calculated by this method is prone to deviation.

**SUMMARY**

In view of this, the embodiments of the present application provide a method for calculating soil mass state data based on a boundary surface plasticity model to solve the problem of low calculation accuracy of boundary surface plasticity models in the prior art.

To achieve the above purpose, the present application provides the following technical schemes:

The embodiments of the present application provide a method for calculating soil mass state data based on boundary surface plasticity model, comprising:

acquiring soil mass state data of a previous increment step, wherein the soil mass state data includes stress data, strain data, and internal variable data;

calculating a first soil mass increment of a current sub-increment step based on the soil mass state data, a preset strain increment and an initial sub-increment step length, wherein the first soil mass increment includes a strain increment of the sub-increment step, a first stress increment, and a first internal variable increment;

calculating a second soil mass increment from the soil mass state data and the first soil mass increment, wherein the second soil mass increment includes a strain increment of the sub-increment step, a second stress increment, and a second internal variable increment;

calculating a local truncation error of the current sub-increment step according to the soil mass state data, the first soil mass increment and the second soil mass increment;

when the local truncation error is smaller than a preset error threshold value, obtaining soil mass state data of the current sub-increment step based on the soil mass state data of the previous increment step, the first soil mass increment and the second soil mass increment;

returning to the step of calculating the first soil mass increment, and calculating the soil mass state data of the next sub-increment step based on the soil mass state data of the current sub-increment step, the preset strain increment and the sub-increment step length, wherein recursive calculation is carried out in sequence until the soil mass state data of the last sub-increment step is obtained, and taking the soil mass state data of the last sub-increment step as the soil mass state data of the current increment step.

In some embodiments, the step of calculating a first soil mass increment of a current sub-increment step based on the soil mass state data, a preset strain increment and an initial sub-increment step length comprises:

dividing the preset strain increment according to the initial sub-increment step length to obtain a strain increment step length;

obtaining a strain increment of a sub-increment step based on the strain increment step length and a serial number of a current sub-increment step;

calculating the first stress increment based on the strain increment of the sub-increment step, the stress data and the internal variable data;

calculating the first internal variable increment based on the stress data, the internal variable data, the strain increment of the sub-increment step and a preset hardening rule.

In some embodiments, the step of calculating the first stress increment based on the strain increment of the sub-increment step, the stress data and the internal variable data comprises:

establishing a first stiffness matrix based on the stress data and the internal variable data;

calculating the first stress increment based on the first stiffness matrix and the strain increment of the sub-increment step.

In some embodiments, the step of calculating a second soil mass increment from the soil mass state data and the first soil mass increment comprises:

establishing a second stiffness matrix based on the stress data and the internal variable data as well as the first stress increment and the first internal variable increment;

calculating the second stress increment based on the second stiffness matrix and the strain increment of the sub-increment step;

calculating the second internal variable increment based on the stress data, the internal variable data, the first stress increment, the first internal variable increment, the strain increment of the sub-increment step and a preset hardening rule.

In some embodiments, the step of calculating a local truncation error of the current sub-increment step according to the soil mass state data, the first soil mass increment and the second soil mass increment comprises:

calculating average stress data of the sub-increment step based on the stress data, the first stress increment and the second stress increment;

calculating average internal variable data of the sub-increment step based on the internal variable data, the first internal variable increment and the second internal variable increment;

calculating a first difference value between the first stress increment and the second stress increment and a second difference value between the first internal variable increment and the second internal variable increment, respectively;

calculating a first ratio of the first difference value to the average stress data and a second ratio of the second difference value to the average internal variable data, respectively;

determining the local truncation error based on the maximum ratio selected from the first ratio and the second ratio.

In some embodiments, when the local truncation error is not less than the preset error threshold value, the method further comprises:

adjusting the initial sub-increment step length based on the local truncation error, and returning to the step of calculating the first soil mass increment of the current sub-increment step based on the soil mass state data, a preset strain increment and an initial sub-increment step length, until the local truncation error of the current sub-increment step is less than the preset error threshold value.

In some embodiments, the step of obtaining soil mass state data of the current sub-increment step based on the soil mass state data of the previous increment step, the first soil mass increment and the second soil mass increment comprises:

adding together the strain data and the strain increment of the sub-increment step to obtain the strain data of the current sub-increment step;

calculating a mean value of the stress increment based on the first stress increment and the second stress increment, and adding together the stress data and the mean value of the stress increment to obtain the stress data of the current sub-increment step;

calculating a mean value of the internal variable increment based on the first internal variable increment and the second internal variable increment, and adding together the internal variable data and the mean value of the internal variable increment to obtain the internal variable data of the current sub-increment step.

The embodiments of the present application also provide an apparatus for calculating soil mass state data based on a boundary surface plasticity model, comprising:

an acquisition module, configured to acquire soil mass state data of a previous increment step, wherein the soil mass state data includes stress data, strain data, and internal variable data;

a first calculation module, configured to calculate a first soil mass increment of a current sub-increment step based on the soil mass state data, a preset strain increment and an initial sub-increment step length, wherein the first soil mass increment includes a strain increment of the sub-increment step, a first stress increment, and a first internal variable increment;

a second calculation module, configured to calculate a second soil mass increment from the soil mass state data and the first soil mass increment, wherein the second soil mass increment includes a strain increment of the sub-increment step, a second stress increment, and a second internal variable increment;

a truncation error calculation module, configured to calculate a local truncation error of the current sub-increment step according to the soil mass state data, the first soil mass increment and the second soil mass increment;

an increment calculation module, configured to, when the local truncation error is smaller than a preset error threshold value, obtain soil mass state data of the current sub-increment step based on the soil mass state data of the previous increment step, the first soil mass increment and the second soil mass increment;

a determination module, configured to return to the step of calculating the first soil mass increment, and calculate the soil mass state data of the next sub-increment step based on the soil mass state data of the current sub-increment step, the preset strain increment and the sub-increment step length, wherein recursive calculation is carried out in sequence until the soil mass state data of the last sub-increment step is obtained, and take the soil mass state data of the last sub-increment step as the soil mass state data of the current increment step.

The embodiments of the present application also provide an electronic device, comprising:
a memory and a processor, the memory and the processor are connected in communication with each other, computer instructions are stored in the memory, and the processor is configured to execute the computer instructions to implement the method provided by the embodiments of the present application for calculating soil mass state data based on a boundary surface plasticity model.

The embodiments of the present application also provide a computer readable storage medium configured to store computer instructions, the computer instructions are configured to cause a computer to implement the method provided by the embodiments of the present application for calculating soil mass state data based on a boundary surface plasticity model.

The technical schemes of the present application have the following advantages:
The present application provides a method for calculating soil mass state data based on a boundary surface plasticity model that comprises: acquiring soil mass state data of a previous increment step, wherein the soil mass state data includes stress data, strain data, and internal variable data; calculating a first soil mass increment of a current sub-increment step based on the soil mass state data, a preset strain increment and an initial sub-increment step length, wherein the first soil mass increment includes a strain increment of the sub-increment step, a first stress increment, and a first internal variable increment; calculating a second soil mass increment from the soil mass state data and the first soil mass increment, wherein the second soil mass increment includes a strain increment of the sub-increment step, a second stress increment, and a second internal variable increment; calculating a local truncation error of the current sub-increment step according to the soil mass state data, the first soil mass increment and the second soil mass increment; when the local truncation error is smaller than a preset error threshold value, obtaining soil mass state data of the current sub-increment step based on the soil mass state data of the previous increment step, the first soil mass increment and the second soil mass increment; returning to the step of calculating the first soil mass increment, and calculating the soil mass state data of the next sub-increment step based on the soil mass state data of the current sub-increment step, the preset strain increment and the sub-increment step length, wherein recursive calculation is carried out in sequence until the soil mass state data of the last sub-increment step is obtained, and taking the soil mass state data of the last sub-increment step as the soil mass state data of the current increment step. By analyzing a local truncation error of the current sub-increment step near the stress reversal point, and adjusting the sub-increment step length based on the analysis results to ensure that the local truncation error is maintained within a certain accuracy range, the present application makes the position of the stress reversal point be captured more accurately, and further improves the calculation accuracy of soil mass state data.

## BRIEF DESCRIPTION OF THE DRAWINGS

In order to illustrate more clearly the technical solutions in the embodiments of the present application or in the prior art, the drawings needed for the description of the embodiments or the prior art will be briefly introduced below. Apparently, the drawings described hereinafter are merely some embodiments of the present application, and a person with ordinary skill in the art may also obtain other drawings according to the described drawings without expenditure of creative labor.

Figure 1 is a flowchart illustrating a method for calculating soil mass state data based on a boundary surface plasticity model in the embodiments of the present application;

Figure 2 is a flowchart of calculating a first soil mass increment of a current sub-increment step according to the embodiments of the present application;

Figure 3 is a flowchart of calculating a first stress increment according to the embodiments of the present application;

Figure 4 is a flowchart of calculating a second soil mass increment according to the embodiments of the present application;

Figure 5 is a flowchart of calculating a local truncation error of the current sub-increment step according to the embodiments of the present application;

Figure 6 is a flowchart of obtaining soil mass state data of the current sub-increment step according to the embodiments of the present application;

Figure 7 is an integration flowchart of a specific implementation scheme according to the embodiments of the present application;

Figure 8 shows the morphological diagram of the boundary surface in the stress space according to the embodiments of the present application;

Figure 9 shows a schematic diagram of the simulation results of the sandy soil shear loading process according to the embodiments of the present application;

Figure 10 shows a schematic diagram of simulating the dynamic tri-axial test of sandy soil according to the embodiments of the present application;

Figure 11 shows a schematic diagram of the results of the dynamic tri-axial test of the sandy soil according to the embodiments of the present application;

Figure 12 is a structural schematic diagram of an apparatus for calculating soil mass state data based on a boundary surface plasticity model in the embodiments of the present application;

Figure 13 is a schematic diagram of an electronic device according to the present application.

**DETAILED DESCRIPTION**

In order to make the objectives, technical schemes and advantages of the embodiments of the present application more clearly understood, the technical solutions in the embodiments of the present application will be clearly and completely described below in conjunction with drawings in the embodiments of the present application. Apparently, the described embodiments are only a part of the embodiments of the present application, not all of the embodiments. Based on the embodiments described in the present application, all other embodiments obtainable by a person with ordinary skill in the art without expenditure of creative labor shall fall within the protection scope of the present application.

According to the embodiments of the present application, there is provided an embodiment of a method for calculating soil mass state data based on a boundary surface plasticity model. It should be noted that the steps shown in the flowchart of the appended drawings can be executed in a computer system such as with a set of computer executable instructions, and although a logical sequence is shown in the flowchart, in some cases, the steps shown or described can be executed in a different sequence than herein.

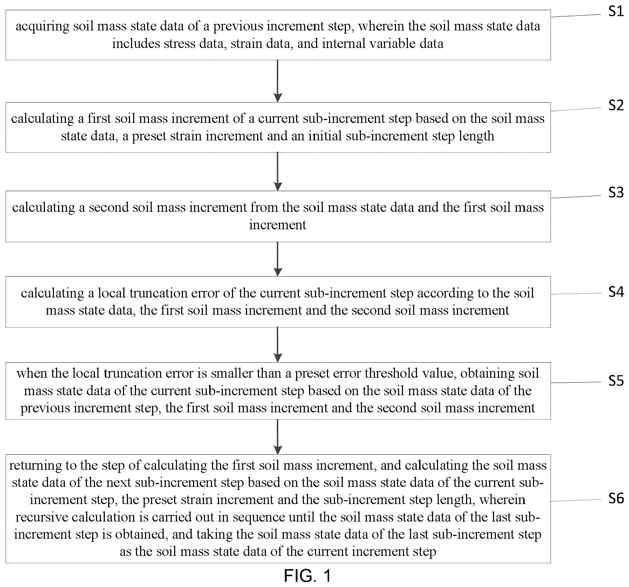

A method for calculating soil mass state data based on a boundary surface plasticity model is provided in this embodiment, which may be used to simulate cyclic loading of soil mass, as shown in FIG. 1, the method for calculating soil mass state data based on a boundary surface plasticity model comprises the flowing steps:

Step S1: acquiring soil mass state data of a previous increment step, wherein the soil mass state data includes stress data, strain data, and internal variable data. Specifically, at the beginning of the $n^{th}$ increment step, the soil mass state after a previous increment step is represented by stress data $\sigma_{(n)}$ strain data $\varepsilon_{(n)}$, and internal variable data $\kappa_{(n)}$ that controls the size and position of the boundary surface, $\sigma_{(n)}$, $\varepsilon_{(n)}$, $\kappa_{(n)}$ will be used as initial conditions for the first sub-increment step, i.e., $\boldsymbol{\sigma}_{(n)}^{(1)} = \boldsymbol{\sigma}_{(n)}$, $\boldsymbol{\varepsilon}_{(n)}^{(1)} = \boldsymbol{\varepsilon}_{(n)}$, $\kappa_{(n)}^{(1)} = \kappa_{(n)}$, wherein, the numerals in the superscript and subscript parentheses represent the serial number of the sub-increment step and the serial number of the increment step, respectively.

Step S2, calculating a first soil mass increment of a current sub-increment step based on the soil mass state data, a preset strain increment and an initial sub-increment step length, wherein the first soil mass increment includes a strain increment of the sub-increment step, a first stress increment, and a first internal variable increment. Specifically, given the preset strain increment $\Delta\varepsilon_{(n)}$ for the $n^{th}$ increment step, $T$ ($0 \leq T \leq 1$) is defined as the "pseudo time" for the calculation of the sub-increment steps to facilitate the division of sub-increment steps, and it is assumed that $T = 0$, $\Delta T^{(1)} = 1$ before the start of the first sub-increment step.

Step S3, calculating a second soil mass increment from the soil mass state data and the first soil mass increment, wherein the second soil mass increment includes a strain increment of the sub-increment step, a second stress increment, and a second internal variable increment.

Step S4, calculating a local truncation error of the current sub-increment step according to the soil mass state data, the first soil mass increment and the second soil mass increment.

Step S5, when the local truncation error is smaller than a preset error threshold value, obtaining soil mass state data of the current sub-increment step based on the soil mass state data of the previous increment step, the first soil mass increment and the second soil mass increment.

Step S6, returning to the step of calculating the first soil mass increment, and calculating the soil mass state data of the next sub-increment step based on the soil mass state data of the current sub-increment step, the preset strain increment and the sub-increment step length, wherein recursive calculation is carried out in sequence until the soil mass state data of the last sub-increment step is obtained, and taking the soil mass state data of the last sub-increment step as the soil mass state data of the current increment step.

By means of the above step S1 to step S6, in the method for calculating soil mass state data based on a boundary surface plasticity model provided in the embodiments of the present application, due to the mutation mechanism of the boundary surface plasticity model and the motion hardening rule, there are many singular points in the mathematical expression thereof, which is not conducive to numerical development. In addition, the boundary surface model of inelastic domain generally judges the stress reversal by recognizing the change between positive and negative signs of a load index, and when the sub-increment step length is large, the position of the stress reversal point calculated by this model is prone to deviation. By analyzing a local truncation error of the current sub-increment step near the stress reversal point, and adjusting the sub-increment step length based on the analysis results to ensure that the local truncation error is maintained within a certain accuracy range, the present application makes the position of the stress reversal point be captured more accurately, and further improves the calculation accuracy of soil mass state data.

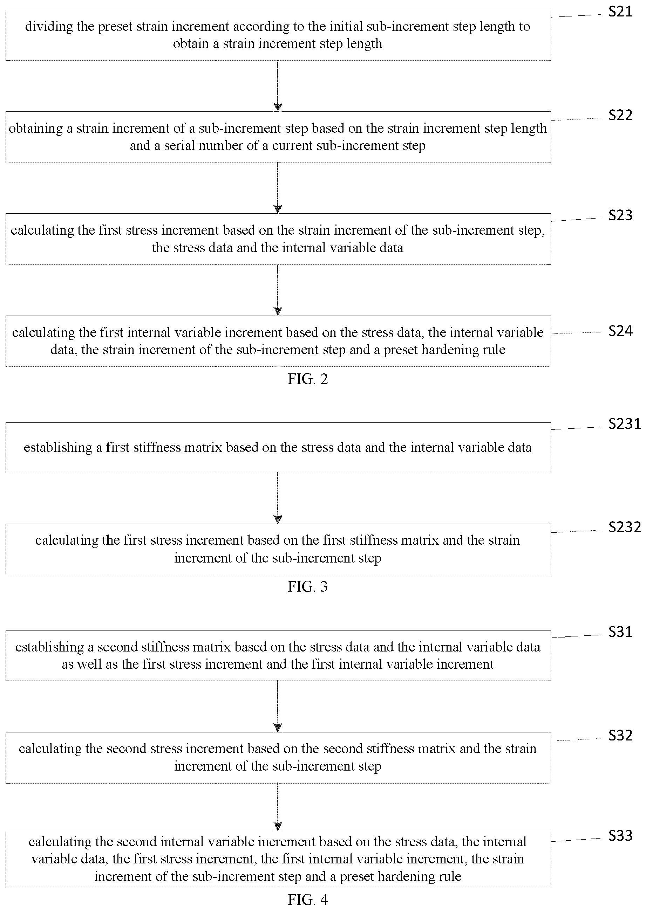

Specifically, in one embodiment, as shown in FIG. 2, the above step S2 includes the following steps:

Step S21, dividing the preset strain increment according to the initial sub-increment step length to obtain a strain increment step length.

Step S22, obtaining a strain increment of a sub-increment step based on the strain increment step length and a serial number of a current sub-increment step. Specifically, for the $k^{th}$ sub-increment step, a strain increment of a sub-increment step can be expressed as $\Delta\varepsilon^{(k)} = \Delta T^{(k)}\Delta\varepsilon_{(n)}$.

Step S23, calculating the first stress increment based on the strain increment of the sub-increment step, the stress data and the internal variable data. Specifically, with the initial conditions $\boldsymbol{\sigma}_{(n)}^{(k)}$、 $\kappa_{(n)}^{(k)}$, of the $k^{th}$ sub-increment step, a stiffness matrix $\mathbf{D}_1^{ep}\left(\boldsymbol{\sigma}_{(n)}^{(k)}, \kappa_{(n)}^{(k)}\right)$ required for the first calculation is calculated, and the first stress increment $\Delta\sigma_1$ in the first calculation can be expressed as $\Delta\boldsymbol{\sigma}_1 = \mathbf{D}_1^{ep}\left(\boldsymbol{\sigma}_{(n)}^{(k)}, \kappa_{(n)}^{(k)}\right)\Delta\boldsymbol{\varepsilon}^{(k)}$ .

Step S24, calculating the first internal variable increment based on the stress data, the internal variable data, the strain increment of the sub-increment step and a preset hardening rule. Specifically, an increment $\Delta\kappa_1$ of an internal variable is calculated based on a hardening rule $\Delta\lambda$ simultaneously, which can be expressed as

$$\Delta\kappa_1 = \Delta\lambda\left(\boldsymbol{\sigma}_{(n)}^{(k)}, \kappa_{(n)}^{(k)}, \Delta\boldsymbol{\varepsilon}^{(k)}\right)$$ .

Specifically, in one embodiment, as shown in FIG. 3, the above step S23 includes the following steps:

Step 231: establishing a first stiffness matrix based on the stress data and the internal variable data. Specifically, with the initial conditions $\boldsymbol{\sigma}_{(n)}^{(k)}$、 $\kappa_{(n)}^{(k)}$ of the $k^{th}$ sub-increment step, a stiffness matrix $\mathbf{D}_1^{ep}\left(\boldsymbol{\sigma}_{(n)}^{(k)}, \kappa_{(n)}^{(k)}\right)$ required for the first calculation is calculated.

Step 232: calculating the first stress increment based on the first stiffness matrix and the strain increment of the sub-increment step. Specifically, the first stress increment $\Delta\sigma_1$ can be expressed as $\Delta\boldsymbol{\sigma}_1 = \mathbf{D}_1^{ep}\left(\boldsymbol{\sigma}_{(n)}^{(k)}, \kappa_{(n)}^{(k)}\right)\Delta\boldsymbol{\varepsilon}^{(k)}$ .

Specifically, in one embodiment, as shown in FIG. 4, the above step S3 includes the following steps:

Step 31: establishing a second stiffness matrix based on the stress data and the internal variable data as well as the first stress increment and the first internal variable increment. Specifically, calculating a second stiffness matrix $\mathbf{D}_2^{ep}\left(\boldsymbol{\sigma}_{(n)}^{(k)} + \Delta\boldsymbol{\sigma}_1, \kappa_{(n)}^{(k)} + \Delta\kappa_1\right)$ based on the stress value $\boldsymbol{\sigma}_{(n)}^{(k)} + \Delta\boldsymbol{\sigma}_1$ and the internal variable value $\kappa_{(n)}^{(k)} + \Delta\kappa_1$ obtained after the first calculation.

Step 32: calculating the second stress increment based on the second stiffness matrix and the strain increment of the sub-increment step.

Step 33: calculating the second internal variable increment based on the stress data, the internal variable data, the first stress increment, the first internal variable increment, the strain increment of the sub-increment step and a preset hardening rule. Specifically, the second stress increment $\Delta\sigma_2$ and the second internal variable increment $\Delta\kappa_2$ can be calculated by the following formulas:

$$\begin{cases} \Delta\boldsymbol{\sigma}_2 = \mathbf{D}_2^{ep}\left(\boldsymbol{\sigma}_{(n)}^{(k)} + \Delta\boldsymbol{\sigma}_1, \kappa_{(n)}^{(k)} + \Delta\kappa_1\right)\Delta\boldsymbol{\varepsilon}^{(k)} \\ \Delta\kappa_2 = \Delta\lambda\left(\boldsymbol{\sigma}_{(n)}^{(k)} + \Delta\boldsymbol{\sigma}_1, \kappa_{(n)}^{(k)} + \Delta\kappa_1, \Delta\boldsymbol{\varepsilon}^{(k)}\right) \end{cases} \quad (1)$$

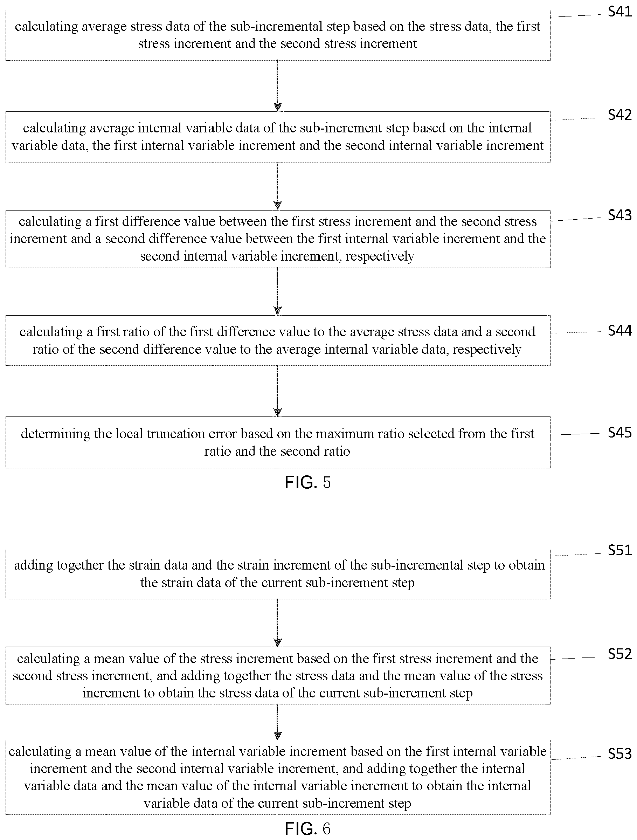

Specifically, in one embodiment, as shown in FIG. 5, the above step S4 includes the following steps:

Step 41: calculating average stress data of the sub-increment step based on the stress data, the first stress increment and the second stress increment.

Step 42: calculating average internal variable data of the sub-increment step based on the internal variable data, the first internal variable increment and the second internal variable increment.

Specifically, the average stress data and the average internal variable data are calculated as follows:

$$\begin{cases} \tilde{\boldsymbol{\sigma}}_{(n)}^{(k+1)} = \boldsymbol{\sigma}_{(n)}^{(k)} + \frac{1}{2}(\Delta\boldsymbol{\sigma}_1 + \Delta\boldsymbol{\sigma}_2) \\ \tilde{\kappa}_{(n)}^{(k+1)} = \kappa_{(n)}^{(k)} + \frac{1}{2}(\Delta\kappa_1 + \Delta\kappa_2) \end{cases} \quad (2)$$

Step 43: calculating a first difference value between the first stress increment and the second stress increment and a second difference value between the first internal variable increment and the second internal variable increment, respectively.

Step 44: calculating a first ratio of the first difference value to the average stress data and a second ratio of the second difference value to the average internal variable data, respectively.

Step 45: determining the local truncation error based on the maximum ratio selected from the first ratio and the second ratio. Specifically, the local truncation error is:

$$R^{(k)} = \max\left\{\frac{1}{2}\frac{\left\|\Delta\boldsymbol{\sigma}_2 - \Delta\boldsymbol{\sigma}_1\right\|}{\left\|\tilde{\boldsymbol{\sigma}}_{(n)}^{(k+1)}\right\|}, \frac{1}{2}\frac{\left|\Delta\kappa_2 - \Delta\kappa_1\right|}{\left|\tilde{\kappa}_{(n)}^{(k+1)}\right|}\right\}.$$

Specifically, in one embodiment, when the local truncation error is not less than the preset error threshold value, the above method for calculating soil mass state data based on a boundary surface plasticity model further comprises: adjusting the initial sub-increment step length based on the local truncation error, and returning to the step of calculating the first soil mass increment of the current sub-increment step based on the soil mass state data, a preset strain increment and an initial sub-increment step length, until the local truncation error of the current sub-increment step is less than the preset error threshold value.

Specifically, calculating the local truncation error $R^{(k)}$ and judging whether the error meets the requirements, and when $R^{(k)}$ is less than the preset error threshold value, which indicates that the calculation of the current sub-increment step converges, proceeding to the next step; when $R^{(k)}$ is greater than the preset error threshold value, it indicates that the calculation of the current sub-increment step does not converge, and the current sub-increment step length should be reduced according to the formula $\varpi = \max\left\{0.9\sqrt{STOL/R^{(k)}}, 0.1\right\}$ and the formula $\Delta T^{(k)} = \max\left\{\varpi\Delta T^{(k)}, \Delta T_{\min}\right\}$, wherein, STOL is the preset error threshold value, $\Delta T_{\min}$ is the minimum sub-increment step length, and simultaneously returning to the step of calculating the first soil mass increment of the current sub-increment step based on the soil mass state data, a preset strain increment and an initial sub-increment step length for recalculation of this sub-increment step. Through this process, the position of the stress reversal point can be captured more accurately, and the calculation accuracy of soil mass state data can be improved.

Specifically, when the calculation of the current sub-increment step converges, setting $T = T + \Delta T^{(k)}$, and stress data and internal variable data are updated according to the following formula (3). It should be noted that, although $\boldsymbol{\sigma}_{(n)}^{(k+1)}$, $\kappa_{(n)}^{(k+1)}$ in formula (3) have the same expression as $\tilde{\boldsymbol{\sigma}}_{(n)}^{(k+1)}$, $\tilde{\kappa}_{(n)}^{(k+1)}$ in formula (2), $\boldsymbol{\sigma}_{(n)}^{(k+1)}$, $\kappa_{(n)}^{(k+1)}$ are used to transfer soil mass state between respective sub-increment steps, and $\boldsymbol{\sigma}_{(n)}^{(k+1)}$, $\kappa_{(n)}^{(k+1)}$ are calculated only when trail calculation of the current sub-increment step satisfies the error requirement; whereas are used to temporarily store the stress data and internal variable data obtained by trial calculation in the sub-increment step, and when the above trial calculation does not satisfy the error requirement, re-calculation will be performed to obtain new $\tilde{\boldsymbol{\sigma}}_{(n)}^{(k+1)}$, $\tilde{\kappa}_{(n)}^{(k+1)}$ ;

$$\begin{cases} \boldsymbol{\sigma}_{(n)}^{(k+1)} = \boldsymbol{\sigma}_{(n)}^{(k)} + \frac{1}{2}(\Delta\boldsymbol{\sigma}_1 + \Delta\boldsymbol{\sigma}_2) \\ \kappa_{(n)}^{(k+1)} = \kappa_{(n)}^{(k)} + \frac{1}{2}(\Delta\kappa_1 + \Delta\kappa_2) \end{cases} \quad (3)$$

1, when $T < 1$, a next sub-increment step length $\Delta T^{(k+1)}$ will be increased according to a local truncation error of the current sub-increment step to improve calculation efficiency. The scaling ratio $\varpi$ for the sub-increment step length is calculated according the following formula (4):

$$\varpi = \min\left\{0.9\sqrt{STOL/R^{(k)}}, 2\right\} \quad (4)$$

In addition, when trial calculation of a previous sub-increment step does not converge, the next sub-increment step length is controlled to be no larger than the current sub-increment step length, i.e.,

$$F = \sqrt{(\bar{\mathbf{r}} - \boldsymbol{\alpha}):(\bar{\mathbf{r}} - \boldsymbol{\alpha})} - \sqrt{\frac{2}{3}}m = 0 \quad;$$

For making $T \leq 1$ after the next sub-increment step is finished, $\Delta T^{(k+1)}$ also should satisfy

$$T^{(k+1)} = \min\left\{\varpi T^{(k)}, 1 - T^{(k)}\right\} \quad;$$

setting $k = k + 1$, returning to the step of calculating a first soil mass increment of a current sub-increment step based on the soil mass state data, a preset strain increment and an initial sub-increment step length for continued calculation;

2, when $T = 1$, the soil mass state data is updated with the stress data $\boldsymbol{\sigma}_{(n)}^{(k+1)}$, the strain data $\boldsymbol{\varepsilon}_{(n)}^{(k+1)}$, and the internal variable data $\kappa_{(n)}^{(k+1)}$, which are the data obtained after the calculation of the current sub-increment step is finished, i.e., $\boldsymbol{\sigma}_{(n+1)} = \boldsymbol{\sigma}_{(n)}^{(k+1)}$, $\boldsymbol{\varepsilon}_{(n+1)} = \boldsymbol{\varepsilon}_{(n)}^{(k+1)}$, $\kappa_{(n+1)} = \kappa_{(n)}^{(k+1)}$, the $n^{th}$ increment step ends at this point.

Specifically, in one embodiment, as shown in FIG. 6, the above step S5 includes the following steps:

Step S51: adding together the strain data and the strain increment of the sub-increment step to obtain the strain data of the current sub-increment step.

Step S52: calculating a mean value of the stress increment based on the first stress increment and the second stress increment, and adding together the stress data and the mean value of the stress increment to obtain the stress data of the current sub-increment step.

Step S53: calculating a mean value of the internal variable increment based on the first internal variable increment and the second internal variable increment, and adding together the internal variable data and the mean value of the internal variable increment to obtain the internal variable data of the current sub-increment step.

Specifically, the soil mass state data of the current sub-increment step is obtained by adding an average increment on the basis of the soil mass state data, the motion hardening rule contained in the boundary surface plasticity model involves multiple triggering mechanisms of instantaneous mutation, which causes the mathematical expression of the model to be discontinuous before and after the mutation, that is, there are singular points, which will seriously affect the stability of the numerical calculation. By adjusting the sub-increment step length near the stress reversal point, the present application makes the position of the stress reversal point be captured more accurately and improves the calculation accuracy of soil mass state data, and discontinuity would not occur before and after the mutation, so that the stability of the numerical calculation is increased.

Specifically, by adjusting the sub-increment step length near the stress reversal point, the present application makes the position of the stress reversal point be captured more accurately, and further improves the calculation accuracy of soil mass state data.

Specifically, in one embodiment, due to that the soil mass will exhibit high stiffness effects after stress reversal, elastic-plastic constitutive models usually use the assumption of elastic unloading or construct a change mechanism of the plastic modulus field to describe this phenomenon. However, no matter which method is adopted, it will result in the mathematical function of the constitutive model being discontinuous at the stress reversal point. The premise of numerical integration is that the integral curve to be solved is continuous and smooth. Therefore, the explicit integration algorithm needs to accurately locate the position of the stress reversal point before performing the calculation of the sub-increment step, and then solve the soil mass responses before and after the stress reversal respectively. Based on the above analysis, this embodiment also adds the following conditional trigger mechanism:

In the numerical integration of a boundary surface model, the load index $L$ is usually calculated by using a strain increment and $L$ can be expressed as: $L = \mathbf{Z} : d\varepsilon$.

(1) setting $N_u=1$ at the beginning of each increment step, and after trial calculation for the current sub-increment step is finished, calculating a load index $L$ based on $\tilde{\sigma}_{(n)}^{(k+1)}$ , $\tilde{\varepsilon}_{(n)}^{(k+1)}$ obtained by the trial calculation and a strain increment step length $\triangle\varepsilon^{(k)}$ of the current sub-increment step;

(2) when $L \geq 0$ , it indicates that no stress reversal occurs, and the stress update process proceeds to the next step with $N_u$ remaining unchanged; and in the next trial calculation, the constitutive relationship is calculated using the information of the previous stress reversal point;

(3) when $L < 0$ and $N_u < 10$, reducing the current sub-increment step length, $\Delta T^{(k)} = 0.5\Delta T^{(k)}$, $\Delta\varepsilon^{(k)} = 0.5\Delta\varepsilon^{(k)}$, and setting $N_u = N_u +1$, then returning to step (1) to redo the trial calculation for the current sub-increment step;

(4) when $L < 0$ and $N_u \geq 10$, taking $\tilde{\sigma}_{(n)}^{(k+1)}$ , $\tilde{\varepsilon}_{(n)}^{(k+1)}$ as the soil mass state of a stress reversal point so as to update the information of stress reversal point, entering the next calculation step after resetting $N_u = 1$, and calculating based on the updated information of stress reverse point in the next calculation.

The present application is described in detail hereinafter in conjunction with a specific implementation scheme.

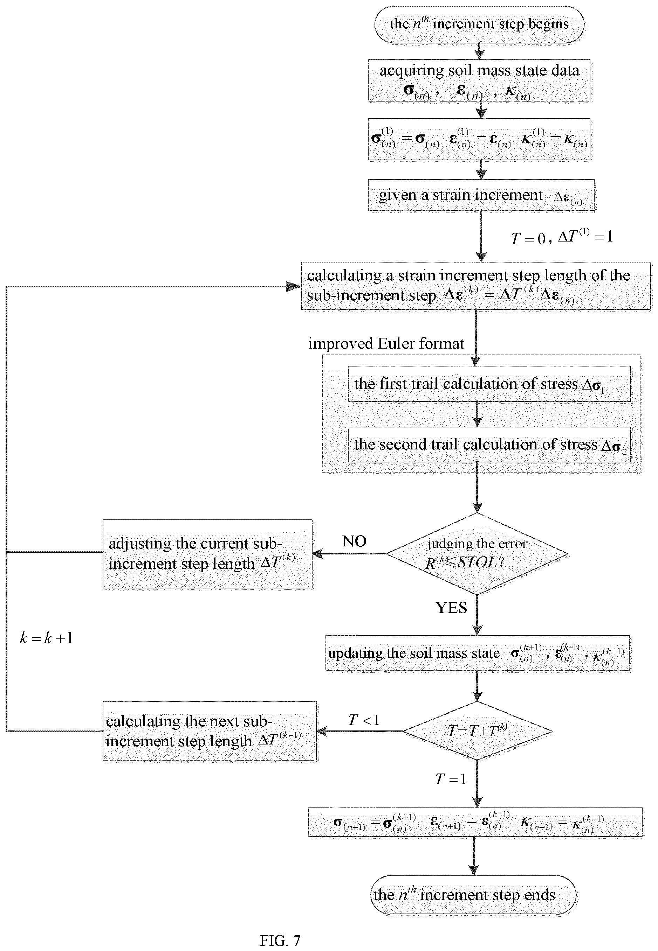

The numerical integration of a certain boundary surface plasticity model is taken as an example, the integration process thereof is shown in FIG. 7. In order to evaluate the performance of this improvement, a soil element model was established in the finite element software, and the accuracy, stability and efficiency of the integration scheme were verified in combination with a tri-axial test.

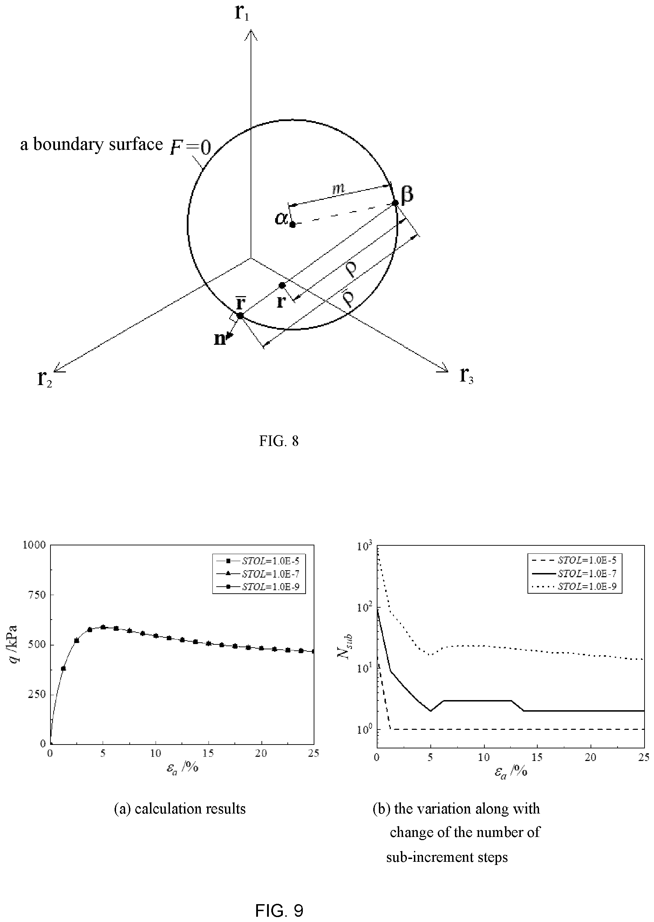

The morphology of a boundary surface in the stress space is shown in FIG. 8, and its mathematical expression is as follows:

$$F = \sqrt{(\overline{\mathbf{r}} - \boldsymbol{\alpha}) : (\overline{\mathbf{r}} - \boldsymbol{\alpha})} - \sqrt{\frac{2}{3}}m = 0$$

wherein, $\overline{\mathbf{r}}$ is the coordinates of the image stress point, $\alpha$ represents the coordinates of the center position of the boundary surface; *m* represents the size of the boundary surface in the stress space. This boundary surface model adopts the assumption of an inelastic domain, and the soil mass flow rule is defined by the following formula:

$$d\mathbf{e}^p = \langle L \rangle \mathbf{n}, \ d\varepsilon_v^p = \langle L \rangle D$$

wherein, *n* is the outer normal vector of the boundary surface, and it is also the direction of plastic deviator strain increment; *L* represents the plastic load index; *D* is the shear expansion ratio, which controls the magnitude of volume strain, and is calculated by the following shear expansion equation:

$$D = \frac{d\varepsilon_v^p}{\sqrt{d\mathbf{e}^p : d\mathbf{e}^p}} = d_0 \left[ \sqrt{\frac{2}{3}} M_c \exp(n_d \psi) \mathbf{n} - \mathbf{r} \right] : \mathbf{n}$$

wherein, $d_0$, $n_d$ are model parameters, $\psi$ is a status parameter, $M_c$ is the critical state stress ratio, and **r** is a deviator stress ratio tensor of the soil mass, which represents the current stress state, $d\varepsilon_v^p$, d$\mathbf{e}^p$ are plastic volumetric strain increment and plastic deviator strain increment, respectively, the plastic load index *L* can be expressed as:

$$L = \frac{1}{K_p} p\mathbf{n} : d\mathbf{r} = \frac{2G(\mathbf{n} : d\mathbf{e}) - (\mathbf{n} : \mathbf{r}) K d\varepsilon_v}{K_p + 2G - (\mathbf{n} : \mathbf{r}) K D}$$

wherein, *p* is the average positive stress; $K_p$ is the plastic modulus; *G* and *K* are shear modulus and bulk modulus, respectively, and d**e** is the deviator strain increment; in order to accurately simulate the variation of plastic deformation of soil mass during cyclic loading, the plastic modulus $K_p$ is calculated using the following formula:

$$K_p = \frac{2}{3} ph \frac{(\mathbf{r}_p - \mathbf{r}) : \mathbf{n}}{(\mathbf{r} - \boldsymbol{\beta}) : \mathbf{n}}$$

wherein, *h* is a hardening parameter, $\mathbf{r}_p$ is the virtual peak deviator stress ratio tensor, which is expressed as follows:

$$\mathbf{r}_p = \sqrt{\frac{2}{3}} M_c \exp(-n_p \psi) \mathbf{n}$$

wherein, $n_p$ is a material constant.

The soil mass stress-strain relationship can be expressed as:

$$d\boldsymbol{\sigma} = \mathbf{D}^{ep} : d\boldsymbol{\varepsilon}$$
$$= \mathbf{D}^e : (d\boldsymbol{\varepsilon} - d\boldsymbol{\varepsilon}^p)$$
$$= \mathbf{D}^e : \left[ d\boldsymbol{\varepsilon} - (\mathbf{n} + \frac{1}{3} D\boldsymbol{\delta}) \langle L \rangle \right]$$

wherein, $\mathbf{D}^e$ is an elastic stiffness matrix, $\mathbf{D}^{ep}$ is an elastic-plastic stiffness matrix, dσ, dε are stress increment and strain

increment, respectively, $\delta$ is a second-order isotropic tensor.

Based on the above boundary surface model, the present application develops a numerical integration process of soil mass constitutive relations, which can be used to simulate the dynamic response generated by cyclic loading of soil mass, and the specific steps thereof are as follows:

(1) at the beginning of the $n^{th}$ increment step, the soil mass state data after a previous increment step is represented together by stress data $\sigma_{(n)}$, strain data $\varepsilon_{(n)}$, and internal variable data $\kappa_{(n)}$ that controls the size and position of the boundary surface, $\sigma_{(n)}$, $\varepsilon_{(n)}$, $\kappa_{(n)}$ will be used as initial conditions for the first sub-increment step, i.e., $\boldsymbol{\sigma}_{(n)}^{(1)} = \boldsymbol{\sigma}_{(n)}$ , $\boldsymbol{\varepsilon}_{(n)}^{(1)} = \boldsymbol{\varepsilon}_{(n)}$ , $\kappa_{(n)}^{(1)} = \kappa_{(n)}$ , wherein, the numerals in the superscript and subscript parentheses represent the serial number of the sub-increment step and the serial number of the increment step, respectively;

(2) given the preset strain increment $\Delta\varepsilon_{(n)}$ for the $n^{th}$ increment step, $T$ ($0 \leq T \leq 1$) is defined as the "pseudo time" for the calculation of the sub-increment step to facilitate the division of sub-increment steps, and it is assumed that $T = 0$ , $\Delta T^{(1)} = 1$ before the start of the first sub-increment step;

(3) calculating a strain increment step length of the sub-increment step, and for the $k^{th}$ sub-increment step, $\Delta\varepsilon^{(k)} = \Delta T^{(k)}\Delta\varepsilon_{(n)}$ ;

(4) with the initial conditions $\boldsymbol{\sigma}_{(n)}^{(k)}$、 $\kappa_{(n)}^{(k)}$ of the $k^{th}$ sub-increment step, a stiffness matrix $\mathbf{D}_1^{ep}\left(\boldsymbol{\sigma}_{(n)}^{(k)}, \kappa_{(n)}^{(k)}\right)$ required for the first trail calculation is calculated, and the stress increment $\Delta\sigma_1$ in the first trail calculation can be expressed as $\Delta\boldsymbol{\sigma}_1 = \mathbf{D}_1^{ep}\left(\boldsymbol{\sigma}_{(n)}^{(k)}, \kappa_{(n)}^{(k)}\right)\Delta\boldsymbol{\varepsilon}^{(k)}$ ;

calculating an internal variable increment $\Delta\kappa_1$ based on a hardening rule $\Delta\lambda$ simultaneously, which can be expressed as $\Delta\kappa_1 = \Delta\lambda\left(\boldsymbol{\sigma}_{(n)}^{(k)}, \kappa_{(n)}^{(k)}, \Delta\boldsymbol{\varepsilon}^{(k)}\right)$ ;

calculating another stiffness matrix $\mathbf{D}_2^{ep}\left(\boldsymbol{\sigma}_{(n)}^{(k)} + \Delta\boldsymbol{\sigma}_1, \kappa_{(n)}^{(k)} + \Delta\kappa_1\right)$ based on the stress value $\boldsymbol{\sigma}_{(n)}^{(k)} + \Delta\boldsymbol{\sigma}_1$ and the internal variable value $\kappa_{(n)}^{(k)} + \Delta\kappa_1$ obtained after the first trail calculation, and performing the second trail calculation, wherein the stress increment $\Delta\sigma_2$ in the second trail calculation and the internal variable increment $\Delta\kappa_2$ in the second trail calculation can be calculated by the following formulas:

$$\begin{cases} \Delta\boldsymbol{\sigma}_2 = \mathbf{D}_2^{ep}\left(\boldsymbol{\sigma}_{(n)}^{(k)} + \Delta\boldsymbol{\sigma}_1, \kappa_{(n)}^{(k)} + \Delta\kappa_1\right)\Delta\boldsymbol{\varepsilon}^{(k)} \\ \Delta\kappa_2 = \Delta\lambda\left(\boldsymbol{\sigma}_{(n)}^{(k)} + \Delta\boldsymbol{\sigma}_1, \kappa_{(n)}^{(k)} + \Delta\kappa_1, \Delta\boldsymbol{\varepsilon}^{(k)}\right) \end{cases}$$

(5) calculating the local truncation error $R^{(k)}$ and judging whether the error meets the requirements, and when $R^{(k)}$ is less than the preset error threshold value $STOL$, which indicates that the calculation of the current sub-increment step converges, proceeding to the next step; when $R^{(k)}$ is greater than $STOL$, it indicates that the calculation of the current sub-increment step does not converge, and the current sub-increment step length $\Delta T^{(k)}$ should be reduced according to the formula $\varpi = \max\left\{0.9\sqrt{STOL/R^{(k)}}, 0.1\right\}$ and the formula $\Delta T^{(k)} = \max\left\{\varpi\Delta T^{(k)}, \Delta T_{\min}\right\}$ , and simultaneously returning to the step of calculating the first soil mass increment of the current sub-increment step for recalculation of this sub-increment step.

(6) when the calculation of the current sub-increment step converges, setting $T = T + \Delta T^{(k)}$, and stress state and internal variable data are updated according to the formula:

$$\begin{cases} \boldsymbol{\sigma}_{(n)}^{(k+1)} = \boldsymbol{\sigma}_{(n)}^{(k)} + \frac{1}{2}(\Delta\boldsymbol{\sigma}_1 + \Delta\boldsymbol{\sigma}_2) \\ \kappa_{(n)}^{(k+1)} = \kappa_{(n)}^{(k)} + \frac{1}{2}(\Delta\kappa_1 + \Delta\kappa_2) \end{cases}$$

(7) when $T < 1$, a next sub-increment step length $\Delta T^{(k+1)}$ will be increased according to a local truncation error of the current sub-increment step to improve calculation efficiency. The scaling ratio $\varpi$ for the sub-increment step length is calculated according the formula

$$\varpi = \min\left\{0.9\sqrt{STOL/R^{(k)}}, 2\right\};$$

In addition, when trial calculation of a previous sub-increment step does not converge, the next sub-increment step length is controlled to be no larger than the current sub-increment step length, i.e., $\varpi = \min\{\varpi, 1\}$;

For making $T \leq 1$ after the next sub-increment step is finished, $\Delta T^{(k+1)}$ also should satisfy

$$T^{(k+1)} = \min\left\{\varpi T^{(k)}, 1 - T^{(k)}\right\};$$

setting $k = k + 1$, and returning to the step of calculating a first soil mass increment of a current sub-increment step for continued calculation;

(8) when $T = 1$, the soil mass state data is updated with the stress data $\boldsymbol{\sigma}_{(n)}^{(k+1)}$, the strain data $\boldsymbol{\varepsilon}_{(n)}^{(k+1)}$, and the internal variable data $\kappa_{(n)}^{(k+1)}$, which are the data obtained after the calculation of the current sub-increment step is finished, i.e., $\boldsymbol{\sigma}_{(n+1)} = \boldsymbol{\sigma}_{(n)}^{(k+1)}$, $\boldsymbol{\varepsilon}_{(n+1)} = \boldsymbol{\varepsilon}_{(n)}^{(k+1)}$, $\kappa_{(n+1)} = \kappa_{(n)}^{(k+1)}$;

the current increment step ends at this point.

Based on the above integration process, the soil mass static tri-axial test is simulated. FIG. 9 shows the simulation results of the sandy soil shear loading process by using the integration scheme of the embodiments. In order to investigate the improved accuracy and efficiency, the monotonic loading process was simulated by using the allowable errors of $10^{-5}$, $10^{-7}$, $10^{-9}$, and the numbers of increment steps of 500, 5000, 50000, respectively. It can be seen from Table 1 that the final values and peak values of deviator stress obtained under different allowable errors are almost the same. This indicates that the soil mass state data calculation method based on the boundary surface plasticity model involved in the present application can correctly integrate the constitutive equation of the soil mass, and can provide sufficiently accurate calculation results when simulating a monotonic loading process.

Table 1 comparison of simulation results of monotonic loading tests

| number of increment steps | allowable error | astringency | maximum number of sub-increment steps | final values of deviator stress/kPa | peak values of deviator stress /kPa | calculation time/s |
|---|---|---|---|---|---|---|
| 500 | $10^{-7}$ | non-convergence | / | / | / | / |
| 5000 | $10^{-5}$ | convergence | 16 | 466.238 | 584.266 | 91 |
| 5000 | $10^{-7}$ | convergence | 92 | 466.246 | 584.272 | 97 |
| 5000 | $10^{-9}$ | convergence | 877 | 466.245 | 584.272 | 105 |
| 50000 | $10^{-7}$ | convergence | 9 | 466.253 | 584.341 | 98 |

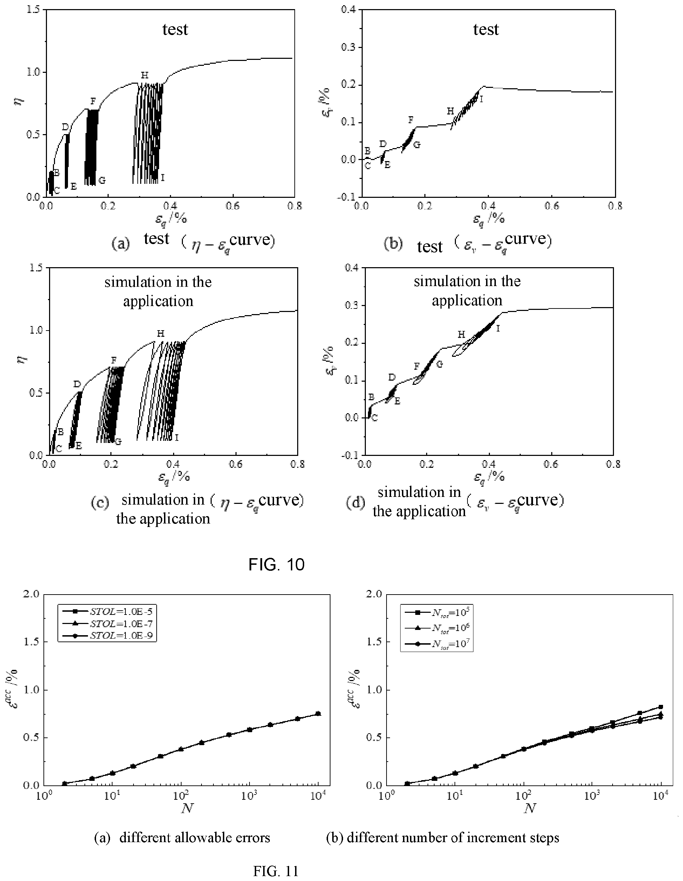

In order to investigate the simulation ability of the integration process in the present application for simulating the cyclic loading of soil mass, this embodiment simulates the dynamic tri-axial test of sandy soil, and the results are shown in FIG. 10. It can be seen that the stress-strain curve calculated by the present application is in good consistence with the test curve, and can reasonably reflect the dynamic response of soil mass at each cyclic loading stage. In order to further investigate the application performance of this scheme when simulating large-cycle cyclic loading, the large-cycle dynamic tri-axial test of sandy soil was simulated, and the results are shown in FIG. 11. It can be seen from the figure that the curves $\varepsilon^{acc}$ - $N$ obtained with different allowable errors are all basically coincide with each other. It can be seen from Table 2 that the calculated cumulative total strain $\varepsilon^{acc}$ is affected by the use of different total numbers $N_{tot}$ of increment steps, that is, by the use of different sizes of increment step lengths. The smaller the increment step length, the higher the accuracy of the calculation, and the smaller the cumulative error when simulating large-cycle cyclic loading, but the influence of the increment step length only gradually appears after the cycle number exceeds 100 cycles. The proposed scheme can not only effectively calculate the cyclic loading of soil mass, but also provide sufficiently high numerical integration accuracy.

Table 2 comparison of simulation results of large-cycle cyclic loading tests

| number of increment steps | allowable error | maximum number of sub-increment steps | $\varepsilon^{acc}$ /% N = $10^2$ | $\varepsilon^{acc}$ /% (N = $10^3$ ) | $\varepsilon^{acc}$ /% (N = $10^4$ ) | calculation time /s |
|---|---|---|---|---|---|---|
| $10^5$ | $10^{-7}$ | 1445 | 0.38485 | 0.60235 | 0.82219 | 1240 |
| $10^6$ | $10^{-5}$ | 216 | 0.37937 | 0.58447 | 0.75057 | 1301 |
| $10^6$ | $10^{-7}$ | 772 | 0.37932 | 0.58451 | 0.75093 | 1343 |
| $10^6$ | $10^{-9}$ | 7705 | 0.37932 | 0.58451 | 0.75093 | 1457 |
| $10^7$ | $10^{-7}$ | 412 | 0.37668 | 0.57252 | 0.71348 | 1406 |

The embodiments of the present application also provide an apparatus for calculating soil mass state data based on a boundary surface plasticity model, the apparatus is used to implement the above embodiments and alternative embodiments, and will not be repeatedly described if it has already been explained. As used below, the term "module" can be a combination of software and/or hardware that implements a predetermined function. Although the apparatus described in the following embodiments are better implemented in software, implementation in hardware or a combination of software and hardware is also possible and envisaged.

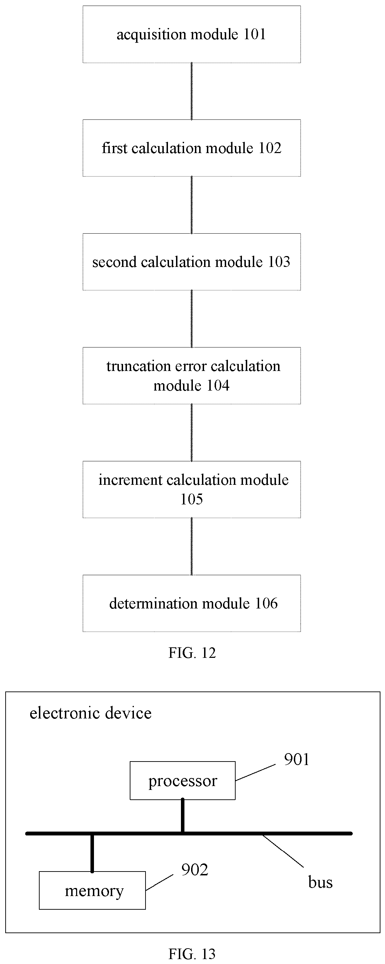

The embodiments of the present application provide an apparatus for calculating soil mass state data based on a boundary surface plasticity model, as shown in FIG. 12, the apparatus comprises:

an acquisition module 101, configured to acquire soil mass state data of a previous increment step, wherein the soil mass state data includes stress data, strain data, and internal variable data, and for the details, please refer to the relevant description of step S1 in the above method embodiments, which will not be repeatedly described herein.

a first calculation module 102, configured to calculate a first soil mass increment of a current sub-increment step based on the soil mass state data, a preset strain increment and an initial sub-increment step length, wherein the first soil mass increment includes a strain increment of the sub-increment step, a first stress increment, and a first internal variable increment, and for the details, please refer to the relevant description of step S2 in the above method embodiments, which will not be repeatedly described herein.

a second calculation module 103, configured to calculate a second soil mass increment from the soil mass state data and the first soil mass increment, wherein the second soil mass increment includes a strain increment of the sub-increment step, a second stress increment, and a second internal variable increment, and for the details, please refer to the relevant description of step S3 in the above method embodiments, which will not be repeatedly described herein.

a truncation error calculation module 104, configured to calculate a local truncation error of the current sub-increment step according to the soil mass state data, the first soil mass increment and the second soil mass increment, and for the details, please refer to the relevant description of step S4 in the above method embodiments, which will not be repeatedly described herein.

an increment calculation module 105, configured to, when the local truncation error is smaller than a preset error threshold value, obtain soil mass state data of the current sub-increment step based on the soil mass state data of the previous increment step, the first soil mass increment and the second soil mass increment, and for the details, please refer to the relevant description of step S5 in the above method embodiments, which will not be repeatedly described herein.

a determination module 106, configured to return to the step of calculating the first soil mass increment, and calculate the soil mass state data of the next sub-increment step based on the soil mass state data of the current sub-increment step, the preset strain increment and the sub-increment step length, wherein recursive calculation is carried out in sequence until the soil mass state data of the last sub-increment step is obtained, and take the soil mass state data of the last sub-increment step as the soil mass state data of the current increment step, and for the details, please refer to the relevant description of step S6 in the above method embodiments, which will not be repeatedly described herein.

The soil state data calculation apparatus based on the boundary surface plasticity model in the present embodiment is presented in the form of functional units, where a unit means an ASIC circuit, a processor and a memory executing one or more software or firmware programs, and/or other devices that can provide the above functions.

The further functional description of each of the above modules is the same as the corresponding embodiments above and will not be repeatedly described herein.

The embodiments of the present application also provide an electronic device, as shown in FIG. 13. The electronic device comprises a processor 901 and a memory 902, wherein, the processor 901 and the memory 902 can be connected by a bus or other means, an example of connection through a bus is shown in FIG. 13. Electronic device is a kind of device that can automatically perform numerical calculation and/or information processing according to pre-set or stored instructions. For example, it can be a rack server, a blade server, a tower server, or a cabinet server (including a standalone server, or a server cluster composed of multiple servers).

The processor 901 can be a Central Processing Unit (CPU). The processor 901 can also be chips, such as other general-purpose processors, Digital Signal Processors (DSPS), Application Specific Integrated Circuits (ASIC), Field-Programmable Gate Array (FPGA) or other programmable logic devices, discrete gates or transistor logic devices, discrete hardware components, or a combination of the various chips mentioned above. The processor 901 is usually used to control the overall operation of the electronic device, such as performing control and processing related to data interaction or communication performed by the electronic device. In this embodiment, the processor 901 is used to run program codes stored in the memory 902 or to process data.

The memory 902 comprises a non-transient computer readable storage medium that can be used to store a non-transient software program, a non-transient computer executable program and modules, such as program instructions/modules corresponding to the method provided by the embodiments of the present application. The processor 901 executes various functional applications and data processing of the processor by running non-transient software programs, instructions and modules stored in the memory 902, so as to realize the method in the above method embodiments.

The memory 902 may include a program storage area and a data storage area, wherein, the program storage area may store an operation system and an application required by at least one function; the data storage area may store the data created by the processor 901, etc. In addition, the memory 902 may include high-speed random access memory and may also include non-transient memory, such as at least one magnetic disk storage device, flash memory device, or other non-transient solid state storage device. In some embodiments, the memory 902 optionally includes memories that are arranged remotely relative to the processor 901 and that can be connected to the processor 901 via a network. Examples of the above network include, but are not limited to, the internet, corporate intranets, local area networks, mobile communication networks, and a combination thereof.

One or more modules stored in the memory 902 implement the method in the above method embodiments when executed by the processor 901.

The specific details of the above electronic device can be understood by referring to the corresponding descriptions and effects in the above method embodiments, and will not be repeatedly described herein.

It is understood by a person skilled in the art that, all or part of the procedures of the methods of the above embodiments can be accomplished by instructing the relevant hardware by a computer program, the program may be stored in a computer readable storage medium, and the program, when executed, may include the procedures of the embodiments of the above various methods. Wherein, the storage media may be a magnetic disk, an optical disc, a Read-Only Memory (ROM), a Random Access Memory (RAM), a Flash Memory, a Hard Disk Drive (HDD), a static random access memory (SRAM), an electrically erasable programmable read-only memory (EEPROM), a programmable read-only memory (PROM) or a Solid-State Drive (SSD); the storage medium may also include a combination of the above kinds of storage media. In some embodiments, the memory 902 may be an internal storage module of an electronic device, such as a hard disk or memory of the electronic device. In some other embodiments, the memory 902 may also be an external storage device for an electronic device, such as a plug-in hard disk, a Smart Media Card (SMC), a Secure Digital (SD) card, and a Flash Card, etc. Of course, the memory 902 may also include both an internal storage module of an electronic device and a storage device external thereto. In this embodiment, the memory 902 is usually configured to store the operation system and various application software installed in an electronic device. In addition, the memory 902 may also be configured to temporarily store various types of data that have been output or will be output.

Although the embodiments of the present application are described in conjunction with the appended drawings, a person skilled in the art can make various modifications and variations without deviating from the spirit and scope of the present application, and such modifications and variations fall within the scope defined by the attached claims.

**Claims**

1.  A method for calculating soil mass state data based on a boundary surface plasticity model, **characterized in** comprising:

    acquiring soil mass state data of a previous increment step, wherein the soil mass state data includes stress data, strain data, and internal variable data;

    calculating a first soil mass increment of a current sub-increment step based on the soil mass state data, a preset strain increment and an initial sub-increment step length, wherein the first soil mass increment includes a strain increment of the sub-increment step, a first stress increment, and a first internal variable increment;

    calculating a second soil mass increment from the soil mass state data and the first soil mass increment, wherein the second soil mass increment includes a strain increment of the sub-increment step, a second stress increment, and a second internal variable increment;

    calculating a local truncation error of the current sub-increment step according to the soil mass state data, the first soil mass increment and the second soil mass increment; the local truncation error

    $$R^{(k)} = \max\left\{\frac{1}{2}\frac{\|\triangle\boldsymbol{\sigma}_2 - \triangle\boldsymbol{\sigma}_1\|}{\|\tilde{\boldsymbol{\sigma}}_{(\mathbf{x})}^{(k+1)}\|}, \frac{1}{2}\frac{|\triangle\mathcal{K}_2 - \triangle\mathcal{K}_1|}{|\tilde{\mathcal{K}}_{(\mathbf{x})}^{(k+1)}|}\right\}$$

    , wherein, $\triangle k_1$ represents the first internal variable increment, $\triangle k_2$ represents the second internal variable increment, $\triangle\sigma_1$ represents the first stress increment, $\triangle\sigma_2$ represents the second stress increment, $\tilde{\boldsymbol{\sigma}}_{(\mathbf{x})}^{(k+1)}$ represents average stress data, $\tilde{\mathcal{K}}_{(\mathbf{x})}^{(k+1)}$ represents average internal variable data;

    when the local truncation error is smaller than a preset error threshold value, obtaining soil mass state data of the current sub-increment step based on the soil mass state data of the previous increment step, the first soil mass increment and the second soil mass increment;

    returning to the step of calculating the first soil mass increment, and calculating the soil mass state data of the next sub-increment step based on the soil mass state data of the current sub-increment step, the preset strain increment and the sub-increment step length, wherein recursive calculation is carried out in sequence until the soil mass state data of the last sub-increment step is obtained, and taking the soil mass state data of the last sub-increment step as the soil mass state data of the current increment step;

    wherein the step of calculating a local truncation error of the current sub-increment step according to the soil mass state data, the first soil mass increment and the second soil mass increment comprises:

    calculating average stress data of the sub-increment step based on the stress data, the first stress increment and the second stress increment;

    calculating average internal variable data of the sub-increment step based on the internal variable data, the first internal variable increment and the second internal variable increment;

    calculating a first difference value between the first stress increment and the second stress increment and a second difference value between the first internal variable increment and the second internal variable increment, respectively;

    calculating a first ratio of the first difference value to the average stress data and a second ratio of the second difference value to the average internal variable data, respectively;

    determining the local truncation error based on the maximum ratio selected from the first ratio and the second ratio;

    the method further comprising a conditional trigger mechanism;

    (1) setting $N_u$=1 at the beginning of each increment step, and after trial calculation for the current sub-increment step is finished, calculating a load index $L_1$ based on $\tilde{\boldsymbol{\sigma}}_{(\mathbf{x})}^{(k+1)}$, $\tilde{\mathcal{K}}_{(\mathbf{x})}^{(k+1)}$ obtained by the trial calculation and a strain increment step length $\triangle\varepsilon^{(k)}$ of the current sub-increment step, wherein, $L_1$=Z:d$\varepsilon$;

    (2) when $L_1 \geq 0$, it indicates that no stress reversal occurs, and the stress update process proceeds to the next step with $N_u$ remaining unchanged; and in the next trial calculation, the constitutive relationship is calculated using the information of the previous stress reversal point;

    (3) when $L_1 < 0$ and $N_u < 10$, reducing the current sub-increment step length, $\triangle T^{(k)} = 0.5\triangle T^{(k)}$, $\triangle\varepsilon^{(k)}$=0.5$\triangle\varepsilon^{(k)}$, and setting $N_u = N_u +1$, then returning to step (1) to redo the trial calculation for the current sub-increment step;
(4) when $L_1 < 0$ and $N_u \geq 10$, taking

$$\tilde{\sigma}_{(n)}^{(k+1)} , \tilde{\varepsilon}_{(n)}^{(k+1)}$$

as the soil mass state of a stress reversal point so as to update the information of stress reversal point, entering the next calculation step after resetting $N_u = 1$, and calculating based on the updated information of stress reverse point in the next calculation;

the mathematical expression of the boundary surface in stress space is as follows:

$$F = \sqrt{(\bar{\mathbf{r}} - \alpha) : (\bar{\mathbf{r}} - \alpha)} - \sqrt{\frac{2}{3}} m = 0$$

wherein, $\bar{\mathbf{r}}$ is the coordinates of the image stress point, $\alpha$ represents the coordinates of the center position of the boundary surface; $m$ represents the size of the boundary surface in the stress space;
the boundary surface model adopts the assumption of an inelastic domain, and the soil mass flow rule is defined by the following formula:

$$d\mathbf{e}^p = \langle L \rangle \mathbf{n}, \ d\varepsilon_v^p = \langle L \rangle D$$

wherein, n is the outer normal vector of the boundary surface, and it is also the direction of plastic deviator strain increment; $L$ represents the plastic load index; $D$ is the shear expansion ratio, which controls the magnitude of volume strain, and is calculated by the following shear expansion equation:

$$D = \frac{d\varepsilon_v^p}{\sqrt{d\mathbf{e}^p : d\mathbf{e}^p}} = d_0 \left[ \sqrt{\frac{2}{3}} M_c \exp(n_d \psi) \mathbf{n} - \mathbf{r} \right] : \mathbf{n}$$

wherein, $d_0$, $n_d$ are model parameters, $\psi$ is a status parameter, $M_c$ is the critical state stress ratio, and $r$ is a deviator stress ratio tensor of the soil mass, which represents the current stress state,

$$d\varepsilon_v^p$$

$d_e^p$ are plastic volumetric strain increment and plastic deviator strain increment, respectively, the plastic load index L can be expressed as:

$$L = \frac{1}{K_p} p\mathbf{n} : d\mathbf{r} = \frac{2G(\mathbf{n} : d\mathbf{e}) - (\mathbf{n} : \mathbf{r}) K d\varepsilon_v}{K_p + 2G - (\mathbf{n} : \mathbf{r}) KD}$$

wherein, $p$ is the average positive stress; $K_p$ is the plastic modulus; $G$ and $K$ are shear modulus and bulk modulus, respectively, and de is the deviator strain increment; in order to accurately simulate the variation of plastic deformation of soil mass during cyclic loading, the plastic modulus $K_p$ is calculated using the following formula:

$$K_p = \frac{2}{3} ph \frac{(\mathbf{r}_p - \mathbf{r}) : \mathbf{n}}{(\mathbf{r} - \beta) : \mathbf{n}}$$

wherein, $h$ is a hardening parameter, $r_p$ is the virtual peak deviator stress ratio tensor, which is expressed as follows:

$$\mathbf{r}_p = \sqrt{\frac{2}{3}} M_c \exp(-n_p \psi) \mathbf{n}$$

wherein, $n_p$ is a material constant.

2. The method for calculating soil mass state data based on a boundary surface plasticity model according to claim 1, **characterized in that**, the step of calculating a first soil mass increment of a current sub-increment step based on the soil mass state data, a preset strain increment and an initial sub-increment step length comprises:

dividing the preset strain increment according to the initial sub-increment step length to obtain a strain increment step length;
obtaining a strain increment of a sub-increment step based on the strain increment step length and a serial number of a current sub-increment step;
calculating the first stress increment based on the strain increment of the sub-increment step, the stress data and the internal variable data;
calculating the first internal variable increment based on the stress data, the internal variable data, the strain increment of the sub-increment step and a preset hardening rule.

3. The method for calculating soil mass state data based on a boundary surface plasticity model according to claim 2, **characterized in that**, the step of calculating the first stress increment based on the strain increment of the sub-increment step, the stress data and the internal variable data comprises:

establishing a first stiffness matrix based on the stress data and the internal variable data;
calculating the first stress increment based on the first stiffness matrix and the strain increment of the sub-increment step.

4. The method for calculating soil mass state data based on a boundary surface plasticity model according to claim 1, **characterized in that**, the step of calculating a second soil mass increment from the soil mass state data and the first soil mass increment comprises:

establishing a second stiffness matrix based on the stress data and the internal variable data as well as the first stress increment and the first internal variable increment;
calculating the second stress increment based on the second stiffness matrix and the strain increment of the sub-increment step;
calculating the second internal variable increment based on the stress data, the internal variable data, the first stress increment, the first internal variable increment, the strain increment of the sub-increment step and a preset hardening rule.

5. The method for calculating soil mass state data based on a boundary surface plasticity model according to claim 1, **characterized in that**, when the local truncation error is not less than the preset error threshold value, the method further comprises:
adjusting the initial sub-increment step length based on the local truncation error, and returning to the step of calculating the first soil mass increment of the current sub-increment step based on the soil mass state data, a preset strain increment and an initial sub-increment step length, until the local truncation error of the current sub-increment step is less than the preset error threshold value.

6. The method for calculating soil mass state data based on a boundary surface plasticity model according to claim 1, **characterized in that**, the step of obtaining soil mass state data of the current sub-increment step based on the soil mass state data of the previous increment step, the first soil mass increment and the second soil mass increment comprises:

adding together the strain data and the strain increment of the sub-increment step to obtain the strain data of the current sub-increment step;

calculating a mean value of the stress increment based on the first stress increment and the second stress increment, and adding together the stress data and the mean value of the stress increment to obtain the stress data of the current sub-increment step;

calculating a mean value of the internal variable increment based on the first internal variable increment and the second internal variable increment, and adding together the internal variable data and the mean value of the internal variable increment to obtain the internal variable data of the current sub-increment step.

7. An apparatus for calculating soil mass state data based on a boundary surface plasticity model, **characterized in** comprising:

an acquisition module, configured to acquire soil mass state data of a previous increment step, wherein the soil mass state data includes stress data, strain data, and internal variable data;

a first calculation module, configured to calculate a first soil mass increment of a current sub-increment step based on the soil mass state data, a preset strain increment and an initial sub-increment step length, wherein the first soil mass increment includes a strain increment of the sub-increment step, a first stress increment, and a first internal variable increment;

a second calculation module, configured to calculate a second soil mass increment from the soil mass state data and the first soil mass increment, wherein the second soil mass increment includes a strain increment of the sub-increment step, a second stress increment, and a second internal variable increment;

a truncation error calculation module, configured to calculate a local truncation error of the current sub-increment step according to the soil mass state data, the first soil mass increment and the second soil mass increment;

$$R^{(k)} = \max \left\{ \frac{1}{2} \frac{\left\| \triangle \boldsymbol{\sigma}_2 - \triangle \boldsymbol{\sigma}_1 \right\|}{\left\| \tilde{\boldsymbol{\sigma}}_{(\boldsymbol{\pi})}^{(k+1)} \right\|} , \frac{1}{2} \frac{\left| \triangle \boldsymbol{\kappa}_2 - \triangle \boldsymbol{\kappa}_1 \right|}{\left| \tilde{\boldsymbol{\kappa}}_{(\boldsymbol{\pi})}^{(k+1)} \right|} \right\}$$

the local truncation error , wherein, $\triangle k_1$ represents the first internal variable increment, $\triangle k_2$ represents the second internal variable increment, $\triangle\sigma_1$ represents the first stress increment, $\triangle\sigma_2$ represents the second stress increment, $\tilde{\boldsymbol{\sigma}}_{(\boldsymbol{\pi})}^{(k+1)}$ represents average stress data, $\tilde{\boldsymbol{\kappa}}_{(\boldsymbol{\pi})}^{(k+1)}$ represents average internal variable data;

wherein, the step of calculating a local truncation error of the current sub-increment step according to the soil mass state data, the first soil mass increment and the second soil mass increment comprises:

calculating average stress data of the sub-increment step based on the stress data, the first stress increment and the second stress increment;

calculating average internal variable data of the sub-increment step based on the internal variable data, the first internal variable increment and the second internal variable increment;

calculating a first difference value between the first stress increment and the second stress increment and a second difference value between the first internal variable increment and the second internal variable increment, respectively;

calculating a first ratio of the first difference value to the average stress data and a second ratio of the second difference value to the average internal variable data, respectively;

determining the local truncation error based on the maximum ratio selected from the first ratio and the second ratio;

an increment calculation module, configured to, when the local truncation error is smaller than a preset error threshold value, obtain soil mass state data of the current sub-increment step based on the soil mass state data of the previous increment step, the first soil mass increment and the second soil mass increment;

a determination module, configured to return to the step of calculating the first soil mass increment, and calculate the soil mass state data of the next sub-increment step based on the soil mass state data of the current sub-increment step, the preset strain increment and the sub-increment step length, wherein recursive calculation is carried out in sequence until the soil mass state data of the last sub-increment step is obtained, and take the soil mass state data of the last sub-increment step as the soil mass state data of the current increment step;

the apparatus further comprising a conditional trigger module configured to:

(1) set $N_u$=1 at the beginning of each increment step, and after trial calculation for the current sub-increment step is finished, calculate a load index $L_1$ based on

$$\tilde{\sigma}_{(n)}^{(k+1)}, \tilde{\mathcal{K}}_{(n)}^{(k+1)}$$

obtained by the trial calculation and a strain increment step length $\triangle\varepsilon^{(k)}$ of the current sub-increment step, wherein, $L_1 = Z:d\varepsilon$;

(2) when $L_1 \geq 0$, it indicates that no stress reversal occurs, and the stress update process proceeds to the next step with $N_u$ remaining unchanged; and in the next trial calculation, the constitutive relationship is calculated using the information of the previous stress reversal point;

(3) when $L_1 < 0$ and $N_u < 10$, reduce the current sub-increment step length, $\Delta T^{(k)} = 0.5\Delta T^{(k)}$, $\varepsilon^{(k)} = 0.5\triangle\varepsilon^{(k)}$, and set $N_u = N_u + 1$, then return to step (1) to redo the trial calculation for the current sub-increment step;

(4) when $L_1 < 0$ and $N_u \geq 10$, take

$$\tilde{\sigma}_{(n)}^{(k+1)}, \tilde{\mathcal{K}}_{(n)}^{(k+1)}$$

as the soil mass state of a stress reversal point so as to update the information of stress reversal point, enter the next calculation step after resetting $N_u = 1$, and calculate based on the updated information of stress reverse point in the next calculation;

the apparatus further comprising a boundary surface module, wherein the mathematical expression of the boundary surface in stress space is as follows:

$$F = \sqrt{(\overline{\mathbf{r}} - \boldsymbol{\alpha}) : (\overline{\mathbf{r}} - \boldsymbol{\alpha})} - \sqrt{\frac{2}{3}}m = 0$$

wherein, $\overline{\mathbf{r}}$ is the coordinates of the image stress point, $\alpha$ represents the coordinates of the center position of the boundary surface; $m$ represents the size of the boundary surface in the stress space;

the boundary surface model adopts the assumption of an inelastic domain, and the soil mass flow rule is defined by the following formula:

$$d\mathbf{e}^p = \langle L \rangle \mathbf{n}, \ d\varepsilon_v^p = \langle L \rangle D$$

wherein, $n$ is the outer normal vector of the boundary surface, and it is also the direction of plastic deviator strain increment; $L$ represents the plastic load index; $D$ is the shear expansion ratio, which controls the magnitude of volume strain, and is calculated by the following shear expansion equation:

$$D = \frac{d\varepsilon_v^p}{\sqrt{d\mathbf{e}^p : d\mathbf{e}^p}} = d_0\left[\sqrt{\frac{2}{3}}M_c\exp(n_d\psi)\mathbf{n} - \mathbf{r}\right] : \mathbf{n}$$

wherein, $d_0$, $n_d$ are model parameters, $\psi$ is a status parameter, $M_c$ is the critical state stress ratio, and $r$ is a deviator stress ratio tensor of the soil mass, which represents the current stress state,

$$d\varepsilon_v^p$$

, $d_e^p$ are plastic volumetric strain increment and plastic deviator strain increment, respectively, the plastic load index $L$ can be expressed as:

$$L = \frac{1}{K_p} p\mathbf{n} : \mathrm{dr} = \frac{2G(\mathbf{n} : \mathrm{de}) - (\mathbf{n} : \mathbf{r}) K \mathrm{d}\varepsilon_v}{K_p + 2G - (\mathbf{n} : \mathbf{r}) KD}$$

wherein, $p$ is the average positive stress; $K_p$ is the plastic modulus; $G$ and $K$ are shear modulus and bulk modulus, respectively, and $de$ is the deviator strain increment; in order to accurately simulate the variation of plastic deformation of soil mass during cyclic loading, the plastic modulus $K_p$ is calculated using the following formula:

$$K_p = \frac{2}{3} ph \frac{(\mathbf{r}_p - \mathbf{r}) : \mathbf{n}}{(\mathbf{r} - \beta) : \mathbf{n}}$$

wherein, $h$ is a hardening parameter, $r_p$ is the virtual peak deviator stress ratio tensor, which is expressed as follows:

$$\mathbf{r}_p = \sqrt{\frac{2}{3}} M_c \exp(-n_p \psi) \mathbf{n}$$

wherein, $n_p$ is a material constant.

8.  An electronic device, **characterized in** comprising:
    a memory and a processor, the memory and the processor are connected in communication with each other, computer instructions are stored in the memory, and the processor is configured to execute the computer instructions to implement the method for calculating soil mass state data based on a boundary surface plasticity model according to any one of claims 1-6.

9.  A computer readable storage medium, configured to store computer instructions, **characterized in that** the computer instructions are configured to cause a computer to implement the method for calculating soil mass state data based on a boundary surface plasticity model according to any one of claims 1-6.

acquiring soil mass state data of a previous increment step, wherein the soil mass state data includes stress data, strain data, and internal variable data — S1

calculating a first soil mass increment of a current sub-increment step based on the soil mass state data, a preset strain increment and an initial sub-increment step length — S2

calculating a second soil mass increment from the soil mass state data and the first soil mass increment — S3

calculating a local truncation error of the current sub-increment step according to the soil mass state data, the first soil mass increment and the second soil mass increment — S4

when the local truncation error is smaller than a preset error threshold value, obtaining soil mass state data of the current sub-increment step based on the soil mass state data of the previous increment step, the first soil mass increment and the second soil mass increment — S5

returning to the step of calculating the first soil mass increment, and calculating the soil mass state data of the next sub-increment step based on the soil mass state data of the current sub-increment step, the preset strain increment and the sub-increment step length, wherein recursive calculation is carried out in sequence until the soil mass state data of the last sub-increment step is obtained, and taking the soil mass state data of the last sub-increment step as the soil mass state data of the current increment step — S6

FIG. 1

dividing the preset strain increment according to the initial sub-increment step length to obtain a strain increment step length — S21

obtaining a strain increment of a sub-increment step based on the strain increment step length and a serial number of a current sub-increment step — S22

calculating the first stress increment based on the strain increment of the sub-increment step, the stress data and the internal variable data — S23

calculating the first internal variable increment based on the stress data, the internal variable data, the strain increment of the sub-increment step and a preset hardening rule — S24

FIG. 2

establishing a first stiffness matrix based on the stress data and the internal variable data — S231

calculating the first stress increment based on the first stiffness matrix and the strain increment of the sub-increment step — S232

FIG. 3

establishing a second stiffness matrix based on the stress data and the internal variable data as well as the first stress increment and the first internal variable increment — S31

calculating the second stress increment based on the second stiffness matrix and the strain increment of the sub-increment step — S32

calculating the second internal variable increment based on the stress data, the internal variable data, the first stress increment, the first internal variable increment, the strain increment of the sub-increment step and a preset hardening rule — S33

FIG. 4

calculating average stress data of the sub-incremental step based on the stress data, the first stress increment and the second stress increment — S41

calculating average internal variable data of the sub-increment step based on the internal variable data, the first internal variable increment and the second internal variable increment — S42

calculating a first difference value between the first stress increment and the second stress increment and a second difference value between the first internal variable increment and the second internal variable increment, respectively — S43

calculating a first ratio of the first difference value to the average stress data and a second ratio of the second difference value to the average internal variable data, respectively — S44

determining the local truncation error based on the maximum ratio selected from the first ratio and the second ratio — S45

FIG. 5

adding together the strain data and the strain increment of the sub-incremental step to obtain the strain data of the current sub-increment step — S51

calculating a mean value of the stress increment based on the first stress increment and the second stress increment, and adding together the stress data and the mean value of the stress increment to obtain the stress data of the current sub-increment step — S52

calculating a mean value of the internal variable increment based on the first internal variable increment and the second internal variable increment, and adding together the internal variable data and the mean value of the internal variable increment to obtain the internal variable data of the current sub-increment step — S53

FIG. 6

the $n^{th}$ increment step begins

acquiring soil mass state data
$\sigma_{(n)}$ , $\varepsilon_{(n)}$ , $\kappa_{(n)}$

$\sigma_{(n)}^{(1)} = \sigma_{(n)}$   $\varepsilon_{(n)}^{(1)} = \varepsilon_{(n)}$   $\kappa_{(n)}^{(1)} = \kappa_{(n)}$

given a strain increment $\Delta\varepsilon_{(n)}$

$T = 0$, $\Delta T^{(1)} = 1$

calculating a strain increment step length of the sub-increment step $\Delta\varepsilon^{(k)} = \Delta T^{(k)}\Delta\varepsilon_{(n)}$

improved Euler format

the first trail calculation of stress $\Delta\sigma_1$

the second trail calculation of stress $\Delta\sigma_2$

adjusting the current sub-increment step length $\Delta T^{(k)}$

NO

judging the error $R^{(k)} \leq STOL$?

YES

$k = k + 1$

updating the soil mass state $\sigma_{(n)}^{(k+1)}$, $\varepsilon_{(n)}^{(k+1)}$, $\kappa_{(n)}^{(k+1)}$

calculating the next sub-increment step length $\Delta T^{(k+1)}$

$T < 1$

$T = T + T^{(k)}$

$T = 1$

$\sigma_{(n+1)} = \sigma_{(n)}^{(k+1)}$   $\varepsilon_{(n+1)} = \varepsilon_{(n)}^{(k+1)}$   $\kappa_{(n+1)} = \kappa_{(n)}^{(k+1)}$

the $n^{th}$ increment step ends

FIG. 7

FIG. 8

(a) calculation results

(b) the variation along with
change of the number of
sub-increment steps

FIG. 9

(a) test ($\eta - \varepsilon_q$ curve)

(b) test ($\varepsilon_v - \varepsilon_q$ curve)

(c) simulation in the application ($\eta - \varepsilon_q$ curve)

(d) simulation in the application ($\varepsilon_v - \varepsilon_q$ curve)

FIG. 10

(a) different allowable errors

(b) different number of increment steps

FIG. 11

acquisition module 101

first calculation module 102

second calculation module 103

truncation error calculation module 104

increment calculation module 105

determination module 106

FIG. 12

electronic device

processor 901

memory 902

bus

FIG. 13

<div align="center">**INTERNATIONAL SEARCH REPORT**</div>

| | |
|---|---|
| | International application No. |
| | **PCT/CN2023/085623** |

**A.    CLASSIFICATION OF SUBJECT MATTER**

G06F30/20(2020.01)i

According to International Patent Classification (IPC) or to both national classification and IPC

**B.    FIELDS SEARCHED**

Minimum documentation searched (classification system followed by classification symbols)

IPC:G06F

Documentation searched other than minimum documentation to the extent that such documents are included in the fields searched

Electronic data base consulted during the international search (name of data base and, where practicable, search terms used)

VEN, CNABS, CNTXT, WOTXT, EPTXT, USTXT, CNKI, IEEE: 土体, 沙土, 边界面塑性, 模型, 增量, 误差, 应力, 应变, sand, bounding surface, model, step, stress, strain

**C.    DOCUMENTS CONSIDERED TO BE RELEVANT**

| Category* | Citation of document, with indication, where appropriate, of the relevant passages | Relevant to claim No. |
|---|---|---|
| PX | CN 114492094 A (CHINA THREE GORGES CORP.) 13 May 2022 (2022-05-13)<br>claims 1-10, and description, paragraphs [0019]-[0053] | 1-9 |
| A | 高源 等 (GAO, Yuan et al.). "适用于饱和砂土循环动力分析边界面塑性模型的显式积分算法 (An Explicit Integration Algorithm of the Bounding-Surface Plasticity Model for Saturated Sand Under Cyclic Loading)"<br>岩土力学 *(Rock And Soil Mechanics),*<br>Vol. 40, No. 10, 31 October 2019 (2019-10-31), 3951-3957<br>pages 3951-3957 | 1-9 |
| A | CN 113189301 A (CHANGSHA UNIVERSITY OF SCIENCE AND TECHNOLOGY) 30 July 2021 (2021-07-30)<br>entire document | 1-9 |
| A | CN 113779775 A (CHINA THREE GORGES CORP.) 10 December 2021 (2021-12-10)<br>entire document | 1-9 |
| A | KR 20020021390 A (KIM YONG SUNG) 20 March 2002 (2002-03-20)<br>entire document | 1-9 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **08 June 2023** | **19 June 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
**Information on patent family members**

International application No.

**PCT/CN2023/085623**

| Patent document cited in search report | | | Publication date (day/month/year) | Patent family member(s) | | | Publication date (day/month/year) |
|---|---|---|---|---|---|---|---|
| CN | 114492094 | A | 13 May 2022 | None | | | |
| CN | 113189301 | A | 30 July 2021 | None | | | |
| CN | 113779775 | A | 10 December 2021 | None | | | |
| KR | 20020021390 | A | 20 March 2002 | KR | 100470788 | B1 | 09 March 2005 |

Form PCT/ISA/210 (patent family annex) (July 2022)

**EP 4 287 061 A1**

**Patent documents cited in the description**

- CN 202210401552